# EUROPEAN PATENT APPLICATION

(11) **EP 4 292 583 A1**
(43) Date of publication of application: **20.12.2023**
(21) Application number: 22752828.8
(22) Date of filing: 10.02.2022
(51) Int. Cl.: A61K 9/08, A61K 38/26, A61K 45/00, A61K 47/12, A61K 47/18, A61K 47/32, A61K 47/38, A61P 3/10, A61P 43/00

(54) **COMPOSITION IN WHICH ABSORBABILITY OF POORLY-ABSORBABLE DRUG IS IMPROVED**

(30) Priority: 12.02.2021 JP 2021021288
(71) Applicant: MEDRx Co., Ltd., Higashikagawa-shi Kagawa 769-2712 (JP)
(72) Inventor: ISHIDA, Tatsuhiro, Tokushima-shi, Tokushima 770-0806 (JP); TATSUMI, Noboru, Higashikagawa-shi, Kagawa 769-2712 (JP); NAKAE, Takashi, Higashikagawa-shi, Kagawa 769-2712 (JP); MIWA, Yasushi, Higashikagawa-shi, Kagawa 769-2712 (JP); HAMAMOTO, Hidetoshi, Higashikagawa-shi, Kagawa 769-2712 (JP)
(74) Representative: Vossius & Partner Patentanwälte Rechtsanwälte mbB
(86) International application number: PCT/JP2022/005412
(87) International publication number: WO 2022/173006

(57) **Abstract**

The present invention provides a composition comprising an ionic liquid and a poorly absorbable drug, wherein the ionic liquid is prepared from an anion and a cation, the anion is an organic acid having 3 to 7 carbon atoms, and the cation is selected from the group consisting of arginine, meglumine, trometamol and diethanolamine, which remarkably improves the absorbability of the poorly absorbable drug.

## Description

### TECHNICAL FILED

The present invention relates to a composition with the improved absorbability of a poorly absorbable drug. Specifically, the present invention relates to a composition comprising a poorly absorbable drug and an ionic liquid in which the intestinal absorbability of the poorly absorbable drug is remarkably improved.

### BACKGROUND ART

Oral administration of a drug is non-invasive, easy to take and drink the drug, mild in the action and effect thereof, and low in cost. A preparation for oral administration is the administration form of a drug that is most acceptable to patients. Also, the preparation for oral administration has been the most widely used in recent years.

Oral administration may be strictly limited due to the chemical and physical barriers in the body. There are the chemical and physical barriers such as extreme pH in the gastrointestinal tract, the exposure to powerful digestive enzymes, and the impermeability of the gastrointestinal tract to an active agent. Biopharmaceutical products such as calcitonin and insulin are often difficult to be administered due to poor intestinal absorbability thereof even when they produce any pharmaceutical effects.

Most of such compounds with poor intestinal absorbability have been developed as injectable preparations. On the other hand, the administration by injection is more physically and mentally damaging for patients as compared to oral administration.

Various intestinal absorption-enhancing technologies have been recently used to develop a preparation for oral administration of a biopharmaceutical product, and the world's first preparation for oral administration of a biopharmaceutical product (Drug Name: Semaglutide, Trade Name: Rybelsus^{®}) was marketed in 2019 and the preparation for oral administration (Drug Name: Octreotide, Trade Name: Mycapssa^{®}) was marketed in 2020.

However, the intestinal absorbability of each oral preparations is 1% or less, and thus the poor absorbability leads to an increase in costs. Also, when the amount of a drug in preparation is increased to cover the poor absorbability of the drug, the preparation's safety is affected due to any disadvantages such as side effects. As a result, there are great problems to improve the poor absorbability of drugs.

In order to deliver such drugs with poor absorbability transmucosally, it is essential to avoid the degradation of the drugs by gastrointestinal enzymes as well as to use the DDS technology which allows the drugs to pass through gastrointestinal epithelial cells and reach the systemic circulation. There are high expectations for an absorption enhancer that can improve the biomembrane permeability of a drug without adversely affecting the drug efficacy using it as an additive for preparation.

The absorption enhancer is a compound that enhances the absorption of a drug, which causes the change in the structure thereof by the direct action of the absorption enhancer on the mucous membranes. Many compounds, including surfactants, bile salts, bacterial toxins, chelating agents, and medium-chain enhancers have been studied for their effectiveness in improving the absorbability of a molecule with poor biological membrane permeability using in vitro and in vivo tests.

However, when the effect of improving the bioavailability (biological availability, hereinafter also referred to as "BA") of the absorption enhancer for a drug with high bioactivity such as biopharmaceuticals is weak, this would lead to greater inter-individual variability and less stable therapeutic effects, resulting in an increase in the amount of a drug required for the development of a preparation. Also, it would lead to higher drug prices.

An ionic liquid consisting of a co-crystal of choline (cation) and geranate (anion) and insulin are mixed to prepare CAGE-insulin, and it has been recently studied to determine whether the prepared CAGE-insulin is absorbed in the intestines and produces hypoglycemic effects when administered orally. However, it is thought that the use of such ionic liquid for drugs other than insulin is problematic.

The present inventors have developed various transdermal absorption preparations which achieve more efficient transdermal absorption of a compound such as a drug with small molecular weight, a nucleic acid with middle molecular weight and a peptide with small molecular weight by the use of an ionic liquid prepared from an organic anion and an organic cation which are in a liquid form at ordinary temperature (hereinafter also referred to as "IL(s)") and have found that an ionic liquid optical for each drug can be prepared. However, the absorption mechanism of drugs differs significantly between transdermal absorption and intestinal (transmucosal) absorption, and the improvement in absorption of drugs cannot be expected even if an ionic liquid for transdermal absorption is used as it is. In order to prepare an ionic liquid for gastrointestinal absorption, a new formulation design is required for the beginning for the type and composition of the ionic liquid.

### PRIOR ART DOCUMENTS

### PATENT DOCUMENTS

Patent Document 1: WO 2019/09983
Patent Document 2: JP 2007-77170

### NON-PATENT DOCUMENTS

Non-Patent Document 1: Maher S., et al., Pharmaceutics, 11, E41 (2019)

### SUMMARY OF INVENTION

### (Problem to be Solved by the Invention)

An object of the present invention is to develop a composition comprising a poorly absorbable drug in which the absorbability of the poorly absorbable drug is remarkably improved.

### (Means for Solving the Problem)

The present inventors have studied novel ionic liquid composition for enhancing the intestinal absorbability of a poorly absorbable drug, that is, the passive diffusion effect of a drug.

As a model compound of poorly absorbable drug, glucagon-like peptide-1 (GLP-1) agonists, which are one of agents for treating type 2 diabetes, were used. Most of the GLP-1 agonists are used as preparations for subcutaneous injection, and thus have the problem of poor medication compliance. On the other hand, preparations for oral administration have the advantages of being easy to administer, non-invasive and safe. If the GLP-1 agonists can be used as preparations for oral administration, the GLP-1 agonists can have high medication compliance.

The GLP-1 agonists are water-soluble compounds with low absorption from the gastrointestinal tract, but it has been expected that the absorption of each GLP-1 agonist in the gastrointestinal tract is improved using an ionic liquid (IL) as an oral base. The absorption of GLP-1 receptor agonists, Lixisenatide (hereinafter also referred to as "Lix.") (Molecular Weight: 4858.49 (4.86 KDa)) and Semaglutide (hereinafter also referred to as "Sem.") (Molecular Weight: 4111 (4.11 KDa)) in the gastrointestinal tract when they are mixed with ionic liquids was evaluated for preparations comprising various types of ionic liquids. In addition, the ionic liquids for enhancing the intestinal absorption were selected using FITC-labeled dextran (Molecular Weight: about 3000 to 40000), which is a water-soluble polymeric compound and is mainly absorbed by the intercellular pathway, as a model compound. As a result, the present inventors have found that the absorption of poorly absorbable drugs were markedly improved using the preparations comprising an ionic liquid an anion which is an organic acid having 3 to 7 carbon atoms and a cation selected from the group consisting of arginine, meglumine, trometamol and diethanolamine. Based on the findings, the present invention has been completed.

That is, the present invention provides the following embodiments.
[Item 1] A composition comprising an ionic liquid and a poorly absorbable drug, wherein the ionic liquid is prepared from an anion and a cation, the anion is an organic acid having 3 to 7 carbon atoms, and the cation is selected from the group consisting of arginine, meglumine, trometamol, and diethanolamine.
[Item 2] The composition according to the above item 1, wherein the poorly absorbable drug is a water-soluble compound with a molecular weight of 3000 to 20000.
[Item 3] The composition according to the above item 1 or 2, wherein the poorly absorbable drug is GLP-1 agonist.
[Item 4] The composition according to any of the above items 1 to 3, wherein the organic acid is one or more selected from the group consisting of lactic acid, citric acid, tartaric acid, malic acid, succinic acid, and benzoic acid.
[Item 5] The composition according to any of the above items 1 to 4, which further comprises one or more water-soluble macromolecules selected from the group consisting of hydroxypropyl cellulose, hydroxypropyl methylcellulose, and polyvinylpyrrolidone.
[Item 6] An oral preparation comprising the composition according to any of the above items 1 to 5.
[Item 7] A method of improving the intestinal absorbability of a poorly absorbable drug, which comprises adding a composition comprising the poorly absorbable drug into an ionic liquid prepared from an anion which is an organic acid having 3 to 7 carbon atoms and a cation selected from the group consisting of arginine, meglumine, trometamol and diethanolamine.

### (Effects of the Invention)

According to the present invention, even a poorly absorbable drug that is difficult to be administered orally can be prepared as an oral preparation by markedly improving the absorbability of the drug. Also, according to the present invention, preparations with better bioavailability than conventional oral preparations can be prepared.

### BRIEF DESCRIPTION OF THE DRAWINGS

FIG. 1 shows the change over time in the blood level of Lixisenatide (pg/mL) when each preparation of Preparation Examples 1, 4 and 5 comprising Lixisenatide as poorly absorbable drug is administered orally and when the preparation of Comparative Example 1 comprising Lixisenatide as poorly absorbable drug is administered subcutaneously.
FIG. 2 shows the change over time in the blood level of Lixisenatide (pg/mL) when the preparation of Preparation Example 1 comprising Lixisenatide as poorly absorbable drug is administered enterally and when the preparation of Comparative Example 1 comprising Lixisenatide as poorly absorbable drug is administered subcutaneously.
FIG. 3-1 shows the blood level of Lixisenatide (pg/mL) at 1 hour after each preparation of Preparation Examples 1 to 8 and Comparative Example 1 comprising Lixisenatide as poorly absorbable drug is administered enterally.
FIG. 3-2 shows the blood level of Lixisenatide (pg/mL) at 1 hour after each preparation of Preparation Examples 9 to 12 comprising Lixisenatide as poorly absorbable drug is administered enterally.
FIG. 3-3 shows the blood level of Lixisenatide (pg/mL) at 1 hour after each preparation of Preparation Examples 13 to 21 comprising Lixisenatide as poorly absorbable drug is administered enterally.
FIG. 4 shows the blood level of Semaglutide (pg/mL) at 1 hour after each preparation of Preparation Examples 1 to 3 and 22 to 23 comprising Semaglutide as poorly absorbable drug is administered enterally.
FIG. 5 shows the fluorescence intensity of FITC-labeled dextran in serum after each preparation of Preparation Examples 1 to 5 and 24 to 26 comprising FTIC-labeled dextran as poorly absorbable drug is administered orally.
FIG. 6 shows the fluorescence intensity of FITC-labeled dextran in serum after each preparation of Preparation Example 1 comprising any of three types of FITC-labeled dextrans with different average molecular weight (10 kDa, 20 kDa and 40 kDa) as poorly absorbable drug is administered orally. In the bar graph of each dextran, the left bar shows Preparation Example 1 and the right bar shows control.

### DESCRIPTION OF EMBODIMENTS

The present invention relates to a composition comprising a poorly absorbable drug and an ionic liquid in which the intestinal absorbability of the poorly absorbable drug is remarkably improved.

As used herein, the "poorly absorbable drug" includes a drug with poor biomembrane permeability, for example, a drug that is difficult to be absorbed from the gastrointestinal tract due to poor permeability of the mucus layer on the gastrointestinal mucosa, a drug that is difficult to be absorbed due to the interaction with substances present in the mucus layer, a drug that is difficult to be absorbed from the gastrointestinal tract due to poor permeability through the gastrointestinal mucosa, a drug that is difficult to be orally absorbed due to the interaction between the mucus layer of the gastrointestinal tract and the mucous membrane, or a drug that is poorly absorbed by the formation of an insoluble complex with bile acid. Generally, the poorly absorbable drug means a drug that is difficult to deliver the drug when administered orally.

The poorly absorbable drug in the present invention can include, for example, peptide, protein, nucleic acid, and a derivative thereof. Examples thereof include insulin, calcitonin, GLP-1 agonist, angiotensin, vasopressin, desmopressin, LH-RH (Luteinizig Hormone-Releasing Hormone), somatostatin, glucagon, oxytocin, gastrin, cyclosporin, somatomedin, secretin, h-ANP (human Atrial Natriuretic Peptide), ACTH (Adrenocorticotropic Hormone), MSH (Melanocyte-Stimulating Hormone), β-endorphin, muramyl dipeptide, enkephalin, neurotensin, bombesin, VIP (Vasoactive Intestinal Peptide), CCK-8 (Cholecystokinin-8), PTH (Parathyroid Hormone), CGRP (Calcitonin Gene-Related Peptide), TRH (Thyrotropin Releasing Hormone), endothelin, hGH (human Growth Hormone), teriparatide or a cytokine such as interleukin, interferon, colony-stimulating factor, tumor necrosis factor, and a derivative thereof. The peptide and protein include not only those of natural origin but also a pharmacologically active derivative and an analog thereof. For example, the calcitonin used in the present invention includes not only a naturally occurring product such as salmon calcitonin, human calcitonin, porcine calcitonin, eel calcitonin and chicken calcitonin but also an analog thereof such as genetic recombinant thereof. Also, the insulin includes not only human insulin, porcine insulin, and bovine insulin but also an analog thereof such as genetic recombinant thereof.

The nucleic acid is a macromolecule and have a polyanion structure in which phosphodiester structures with negative charges are continuous. Hence, the nucleic acid has the feature that is hard to permeate hydrophobic cell membranes. Examples of the nucleic acid include an antisense nucleic acid (fomivirsen, mipomersen, eteplirsen, nusinersen), Mucugen pegylated with RNA aptamer, SiRNA, decoy nucleic acid, CpG oligonucleotide, and a derivative thereof.

The GLP-1 agonist is an analog of glucagon like peptide-1 (GLP-1), which is an incretin hormone secreted in the body, and acts on GLP-1 receptors to increase cAMP and promote insulin secretion depending on the glucose concentration. Examples of the GLP-1 agonist include semaglutide, dulaglutide, lixisenatide, exenatide, liraglutide, and an analog thereof such as genetic recombinant thereof.

Also, the poorly absorbable drug includes a compound that is difficult to be orally administered other than peptide, protein and nucleic acid and examples thereof include vancomycin, gentamicin, and remdesivir.

The level of the oral absorbability of the poorly absorbable drug in the present invention should not be particularly limited. A drug that is poorly absorbed in the gastrointestinal tract and does not produce sufficient pharmacological effect when administered orally can produce an expected pharmacological effect by increasing the oral absorbability of the drug. Also, a drug with poor absorbability in the gastrointestinal tract that produces sufficient pharmacological effect when administered orally can reduce the dose of the drug and side effects by further increasing the oral absorbability of the drug.

The term "ionic liquid" in the present invention refers to a Bronsted salt prepared from an anion and a cation, which is in a viscous liquid form at ordinary temperature and is a melting point of 100°C or less. The anion and cation may be prepared by mixing an organic anion such as an organic acid and an organic cation in equimolar amounts or excess amounts at room temperature or with heating. The excess amounts of the organic anion and/or organic cation are preferably within 10-time molar amounts.

The "organic acid" in the present invention is a generic term for acidic organic compounds. Carbon chain(s) in the organic acid may be straight or branched and saturated or unsaturated. An organic carboxylic acid is suitable, but the organic acid may be an organic acid with carboxyl group(s) and an organic acid with hydroxy group(s) in addition to carboxyl group(s). Examples of the organic acid include an organic acid having 3 to 7 carbon atoms. The organic acid is preferably lactic acid, citric acid, tartaric acid, succinic acid, malic acid, and benzoic acid.

The "cation" in the present invention is a cationic organic compound, and examples thereof include an organic amine, an organic quaternary ammonium cation, a basic amino acid, and an amino sugar.

Examples of the organic cation include an organic amine having 4 to 12 carbon atoms. The organic cation is preferably diethanolamine, triethanolamine, arginine, lysine, asparagine, meglumine, and trometamol. Particularly, the organic cation is preferably diethanolamine, arginine, meglumine, and trometamol.

The ionic liquid of the present invention is prepared from a combination of the above organic anion and the above organic cation. The ionic liquid may be an ionic liquid prepared by mixing two or more ionic liquids (including a mixed ionic liquid in which only an organic acid or an organic amine compound is different). Also, the ionic liquid may comprise, for example, 1 to 10 times the amount of water relative to the amount of the ionic liquid.

The composition (preparation) of the present invention may be used in combination with a pharmaceutically acceptable carrier. Examples of the carrier include excipient, coating agent, binder, extender, disintegrant, surfactant, lubricant, diluent, dispersant, buffer, osmotic pressure adjuster, pH adjuster, emulsifier, preservative, stabilizer, antioxidant, colorant, ultraviolet absorber, moisturizer, thickener, activity enhancer, flavoring agent, and deodorant.

The carrier may be used alone, respectively, or two or more of the carriers may be combined in appropriate amounts.

When the poorly absorbable drug is an peptide, a protein and a nucleic acid, a pharmaceutical composition for oral use can be provided as long as the preparation technology, in which the drug is not decomposed and is delivered to the lower part of the digestive tract such as jejunum, ileum, colon and large intestine where digestive enzymes have little effect, is used. Examples of such preparation technology include sustained-release pharmaceutical preparation, colon-released pharmaceutical preparation, timed-release or pulsed-release pharmaceutical preparation, solid dosage form such as tablet, coated tablet, granule, powder and capsule, and liquid dosage form such as elixir, syrup, and suspension.

Also, the composition of the present invention may be formulated into the form of capsule comprising a water-soluble encapsulating agent. The water-soluble encapsulating agent is a substance for forming the film of a capsule. The water-soluble encapsulating agent seals content solution to produce the quality retention ability such as the antioxidation of the content solution, and it can be formulated as a capsule.

When the composition of the present invention is formulated into a capsule, it may be formulated by a water-soluble macromolecule such as hydroxypropyl cellulose, hydroxypropyl methylcellulose, and polyvinylpyrrolidone.

### EXAMPLES

Hereinafter, the present invention is illustrated in Examples to make it easy to understand the present invention. The present invention, however, is not intended to be limited thereto by any means.

In the following Examples, substances such as reagent were obtained from manufactures. The present invention, however, is not intended to be limited thereto by any means.
Lactic Acid (Japanese Pharmacopoeia: Kenei Pharmaceutical Co., Ltd.)
Oleic Acid: Croda Japan K.K.
Malic Acid, Stearic Acid, (L+) Tartaric Acid, Succinic Acid, Citric Acid, Diethanolamine, Trometamol: FUJIFILM Wako Pure Chemical Corporation
Purified Water: Otsuka Pharmaceutical Factory Inc.
Meglumine: Tokyo Chemical Industry Co., Ltd.

### Example 1: Preparation of ionic liquid-containing preparation

Each ingredient was weighted in the amount (molar ratio) shown in Table 1 below and the ingredients were mixed to prepare an ionic liquid formulation. A poorly absorbable drug was then dissolved into the prepared ionic liquid formulation to prepare each ionic liquid preparation of Preparation Examples 1 to 21. Also, the preparation in which a poorly absorbable drug was dissolved into saline was prepared as Comparative Example 1.

In the table, the term "ILs (A)" means ionic liquids with high water solubility and the term "ILs (B)" means ionic liquids with higher lipid solubility compared to ILs (A). The number in each parenthesis is the molar ratio of the reagent.

**[Table 1]**

| | ILs (A) | | ILs (B) | | |
|---|---|---|---|---|---|
| Preparation Example 1 | Lactic acid (5) | Trometamol (1) | | | |
| Preparation Example 2 | Lactic acid (5) | Arginine (1) | | | |
| Preparation Example 3 | Lactic acid (5) | Meglumine (1) | | | |
| Preparation Example 4 | | | Oleic acid (5) | Arginine (1) | |
| Preparation Example 5 | Lactic acid (5) | Trometamol (1) | Oleic acid (5) | Arginine (1) | |
| Preparation Example 6 | Lactic acid (1) | Diethanolamine (1) | | | |
| Preparation Example 7 | Lactic acid (5) | Diethanolamine (1) | | | |
| Preparation Example 8 | Malic acid (5) | Diethanolamine (10) | | | |
| Preparation Example 9 | Lactic acid (1) | Arginine (1) | | | Water (5) |
| Preparation Example 10 | Lactic acid (1) | Meglumine (1) | | | Water (5) |
| Preparation Example 11 | Malic acid (1) | Meglumine (1) | | | Water (5) |
| Preparation Example 12 | Malic acid (1) | Trometamol (1) | | | Water (5) |
| Preparation Example 13 | Benzoic acid (1) | Arginine (1) | | | Water (5) |
| Preparation Example 14 | Benzoic acid (1) | Meglumine (1) | | | Water (5) |
| Preparation Example 15 | Tartaric acid (1) | Arginine (1) | | | Water (5) |
| Preparation Example 16 | Tartaric acid (1) | Meglumine (1) | | | Water (5) |
| Preparation Example 17 | Succinic acid (1) | Meglumine (1) | | | Water (5) |
| Preparation Example 18 | Succinic acid (0.5) Malic acid (0.5) | Trometamol (1) | | | Water (5) |
| Preparation Example 19 | Lactic acid (2) | Arginine (1) | | | Water (5) |
| Preparation Example 20 | Lactic acid (2) | Meglumine (1) | | | Water (5) |
| Preparation Example 21 | Lactic acid (2) | Trometamol (1) | | | Water (5) |
| Comparative Example 1 | Saline | | | | |

### Example 2: Evaluation of blood level of poorly absorbable drug when administered orally

In this study, Lixisenatide (purchased from SCRUM Inc.) was used as poorly absorbable drug and the blood level of the drug after oral administration was evaluated according to the following procedure.

According to the method of Example 1, the preparations of Examples 1, 4 and 5 comprising Lixisenatide as poorly absorbable drug were prepared. Specifically, the ingredients were mixed in the amounts (molar ratios) shown in the above Table 1 to prepare each ionic liquid formulation. Lixisenatide (1.4 mg) was added into the prepared ionic liquid formulation (0.5 mL) and the mixture was heated and dissolved at 65°C for 10 minutes to prepare each preparation.

When Lixisenatide was mixed with an ionic liquid and checked the state under a microscope, it was determined that Lixisenatide was dissolved because the crystal of Lixisenatide disappeared by heating.

As a control, the preparation of Comparative Example 1 in which Lixisenatide was dissolved into saline was prepared.

### (Test Method)

As experimental animals, BALB/c mice (5-week age, male) (purchased from Japan SLC (Shizuoka, JAPAN)) were used.

The BALB/c mice were fasted overnight, and the mice were given each preparation of Preparation Examples 1, 4 and 5 comprising Lixisenatide in the adjusted amount (12.5 mg/kg as the Lixisenatide amount) orally or were given the preparation of Comparative Example 1 (10 µg/kg as the Lixisenatide amount) subcutaneously. Blood was then collected from the inferior vena cava of each mouse using a syringe containing heparin sodium at 1, 2 and 4 hours after the administration of each preparation, the collected blood was centrifuged (4°C, 10 min, 3,000 x g) to collect the plasma, and the collected plasma was frozen and stored at - 80°C. The plasma was quantified by Lixisenatide EIA Kit (Phoenix Pharmaceuticals Inc., CA, USA).

Each preparation of Preparation Examples 1, 4 and 5 was orally administered and the blood level of Lixisenatide was measured. The relative BA (0-4 h) of each Preparation Example was calculated using the AUC in the blood level curve of Comparative Example 1 as 100%.

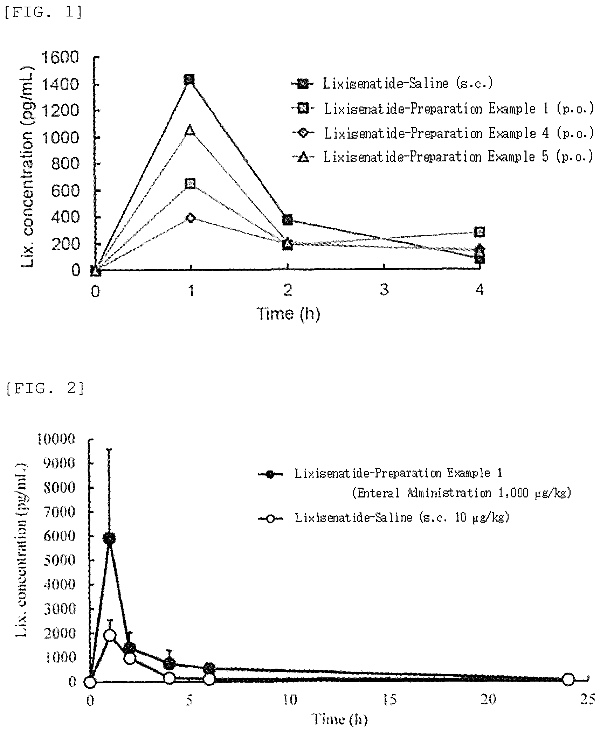

The result is shown in FIG. 1. FIG. 1 shows that he preparations of Preparation Examples 1 and 5 had the higher blood level of Lixisenatide at 1 hour after the administration, and thus Lixisenatide was transferred into blood by the combination of oral administration and an ionic liquid. On the other hand, it is shown that Preparation Example 4 had the low blood transfer level of Lixisenatide. The BA values of each preparation were 0.046% in Preparation Example 1, 0.032% in Preparation Example 4 and 0.058% in Preparation Example 5 and the values were low because of the degradation of Lixisenatide in the stomach.

### Example 3: Evaluation of blood level of poorly absorbable drug when administered enterally

As experimental animals, BALB/c mice (5-week age, male) (purchased from Japan SLC (Shizuoka, JAPAN)) were used.

The BALB/c mice were fasted overnight, the abdomens of the mice were cut open, and then the mice were given the preparation of Preparation Example 1 comprising Lixisenatide in the adjusted amount (1 mg/kg as the Lixisenatide amount, 10-fold dilution of the ionic liquid of Preparation Example 1) as poorly-absorbable drug enterally into the intestinal region between the duodenum and jejunum as well as given the preparation of Comparative Example 1 comprising Lixisenatide (10 µg/kg as the Lixisenatide amount) as poorly-absorbable drug subcutaneously.

Blood was then collected from the inferior vena cava of each mouse using a syringe containing heparin sodium at 1, 2, 4, 6 and 24 hours after the administration of each preparation, the collected blood was centrifuged (4°C, 10 min, 3,000 x g) to collect the plasma, and the collected plasma was frozen and stored at -80°C. The plasma was quantified by Lixisenatide EIA Kit (Phoenix Pharmaceuticals Inc., CA, USA).

The result is shown in FIG. 2. FIG. 2 shows that in the enteral administration of the preparation of Preparation Example 1, the blood level of Lixisenatide reached a peak (5,900 pg/mL) at 1 hour after the administration and then gradually decreased, and Lixisenatide disappeared from the blood at 24 hours after the administration. The relative BA value of Preparation Example 1 calculated using the AUC of the blood level curve of Comparative Example 1 as 100% was about 2.9%. The value greatly exceeded the BA value of Preparation Example 1 when administered orally (0.046%). The result of the comparative study on the migration ability of the poorly absorbable drug into blood in various ionic liquid formulations when administered enterally showed that it is possible to prepare an ionic liquid suitable for intestinal absorption to apply the ionic liquid as a preparation for oral administration.

### Example 4: Evaluation of migration ability of poorly absorbable drug into blood when administered enterally (1)

According to the method of Example 1, the preparations of Preparation Examples 1 to 21 comprising Lixisenatide as poorly absorbable drug. Specifically, the ingredients were mixed in the amounts (molar ratios) shown in the above Table 1 to prepare each ionic liquid formulation, Lixisenatide (1.4 mg) was added into the prepared ionic liquid formulation (0.5 mL) and the mixture was heated and dissolved at 65°C for 10 minutes to prepare each preparation. The migration ability of Lixisenatide into blood when administered enterally was studied for the preparations of Preparation Examples 1 to 21. As experimental animals, ICR mice (8- to 9-week age, male) (purchased from Japan SLC (Shizuoka, JAPAN)) were used.

The ICR mice were fasted overnight, the abdomens of the mice were cut open, and then the mice were given each preparation of Preparation Examples 1 to 21 comprising Lixisenatide in the adjusted amount (1 mg/kg as the Lixisenatide amount) enterally into the intestinal region between the duodenum and jejunum. Blood was collected from the inferior vena cava of each mouse using a syringe containing heparin sodium at 1 hour after the administration of each preparation, the collected blood was centrifuged (4°C, 10 min, 3,000 x g) to collect the plasma, and the collected plasma was frozen and stored at -80°C. The plasma was quantified by Lixisenatide EIA Kit (Phoenix Pharmaceuticals Inc., CA, USA).

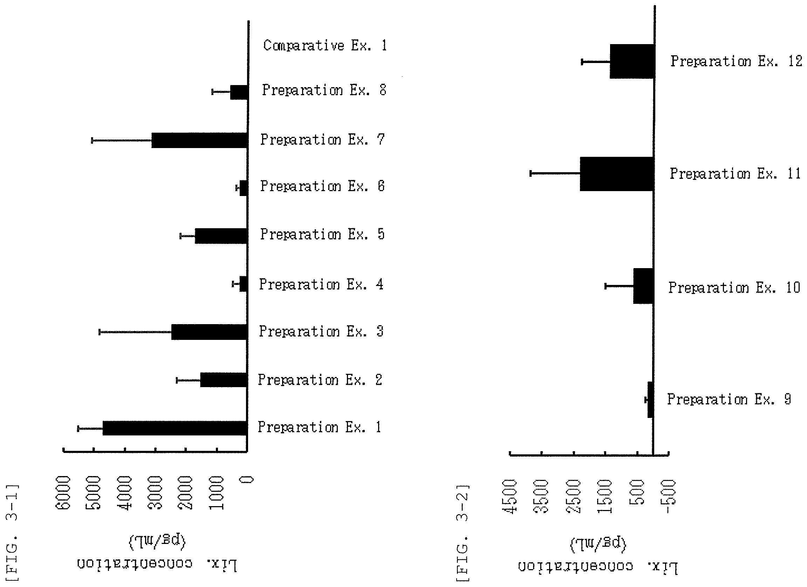

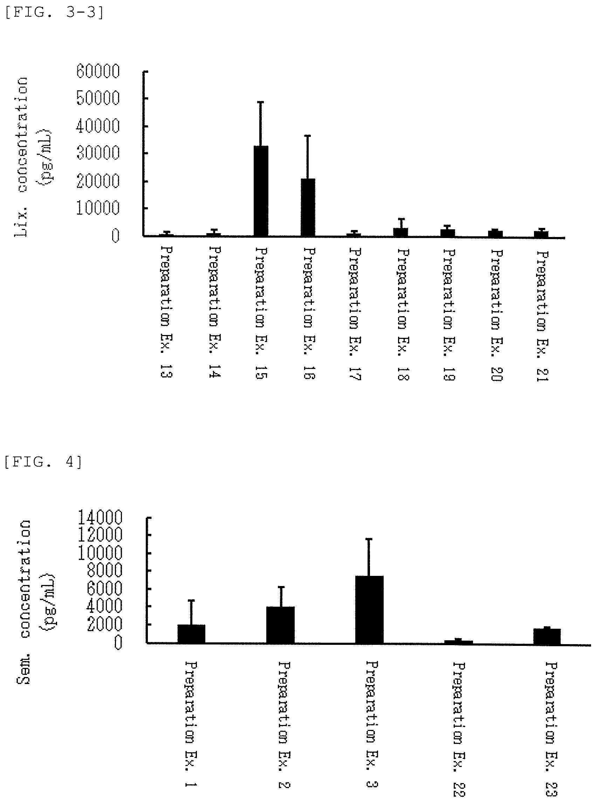

The result is shown in FIG. 3-1 to FIG. 3-3. FIG. 3-1 shows that the preparations of Preparation Examples 1 to 3, 5 and 7 comprising an ionic liquid with lactic acid had the higher migration ability of Lixisenatide into blood. On the other hand, it is shown that the preparations of Preparation Examples 6 and 9 to 10 comprising an ionic liquid with a low ratio of lactic acid had the lower migration ability of Lixisenatide into blood. The result suggests that the amount of lactic acid is important for the migration ability of a poorly absorbable drug into blood.

It is shown that the migration ability of Lixisenatide into blood in the preparations of Preparation Examples 11 and 12 comprising an ionic liquid with malic acid was better, and the preparations of Preparation Examples comprising an ionic liquid with tartaric acid showed particularly high migration ability of Lixisenatide into blood. In addition, not only the preparations of Preparation Examples 13 and 14 comprising an ionic liquid with benzoic acid but also the preparations of Preparation Examples 17 and 18 comprising an ionic liquid with succinic acid showed good migration ability of Lixisenatide into blood. On the other hand, the preparation of Preparation Example 4 comprising only an ionic liquid with oleic acid showed poor migration ability of Lixisenatide into blood.

The result suggests that the ionic liquids can enhance the intestinal absorbability of poorly absorbable drugs because the blood level of poorly absorbable Lixisenatide is markedly increased when administered enterally with an ionic liquid comprising an organic acid in a certain amount.

### Example 5: Evaluation of migration ability of poorly absorbable drug into blood when administered enterally (2)

In this study, Semaglutide (purchased from SCRUM Inc.) was used as poorly absorbable drug and the migration ability of the drug into blood was studied according to the following procedures.

According to the method of Example 1, the preparations of Preparation Examples 1 to 3 and 22 to 23 comprising Lixisenatide as poorly absorbable drug were prepared. Specifically, the ingredients were mixed in the amounts (molar ratios) shown in Table 2 below to prepare each ionic liquid formulation, Semaglutide (1.4 mg) was added into the prepared ionic liquid formulation (0.5 mL), and the mixture was heated and dissolved at 65°C for 10 minutes to prepare each preparation.

When Semaglutide was mixed with an ionic liquid and checked the state under a microscope, it was determined that Semaglutide was dissolved because the crystal of Semaglutide disappeared by heating.

**[Table 2]**

| | ILs (A) | | ILs (B) | |
|---|---|---|---|---|
| Preparation Example 1 | Lactic acid (5) | Trometamol (1) | | |
| Preparation Example 2 | Lactic acid (5) | Arginine (1) | | |
| Preparation Example 3 | Lactic acid (5) | Meglumine (1) | | |
| Preparation Example 22 | | | Oleic acid (5) | Meglumine (1) |
| Preparation Example 23 | | | Oleic acid (5) | Trometamol (1) |

As experimental animals, ICR mice (8- to 9-week age, male) (purchased from Japan SLC (Shizuoka, JAPAN)) were used.

The ICR mice were fasted overnight, the abdomens of the mice were cut open, and then the mice were given each preparation of Preparation Examples 1 to 3 and 22 to 23 comprising Semaglutide in the adjusted amount (0.4 mg/kg as the Semaglutide amount) enterally into the intestinal region between the duodenum and jejunum. Blood was collected from the inferior vena cava of each mouse using a syringe containing heparin sodium at 1 hour after the administration of each preparation, the collected blood was added into a tube containing EDTA and mixed well. The tube was centrifuged (4°C, 10 min, 3,000 x g) to collect the plasma, and the collected plasma was frozen and stored at -80°C. The plasma was quantified by Semaglutide EIA Kit (Peninsula Laboratories International Inc., CA, USA).

The result is shown in FIG. 4. FIG. 4 shows that the preparations of Preparation Examples 1 to 3 had the high blood levels of Semaglutide, and the preparation of Preparation Example 3 had the particularly high blood level of Semaglutide. On the other hand, the preparations of Preparation Example 22 comprising a combination of oleic acid and meglumine and Preparation Example 23 comprising a combination of oleic acid and trometamol had the lower blood levels of Semaglutide.

### Example 6: Evaluation of migration ability of poorly absorbable drug into blood when administered orally (1)

In this study, the compound with poor intestinal absorbability, dextran was used and the migration ability of the drug into blood was studied according to the following procedures. FITC-labeled dextran with an average molecular weight of 3000 to 5000 were purchased from Sigma-Aldrich (MO, USA) .

According to the method of Example 1, the preparations of Preparation Examples 1 to 5 and 24 to 26 comprising FITC-labeled dextran as poorly absorbable drug were prepared. Specifically, the ingredients were mixed in the amounts (molar ratios) shown in Table 3 below to prepare each ionic liquid formulation, and FITC-labeled dextran (2.5 mg) was added and dissolved into the prepared ionic liquid formulation (1 mL) to prepare each preparation.

**[Table 3]**

| | ILs (A) | | ILs (B) | |
|---|---|---|---|---|
| Preparation Example 1 | Lactic acid (5) | Trometamol (1) | | |
| Preparation Example 2 | Lactic acid (5) | Arginine (1) | | |
| Preparation Example 3 | Lactic acid (5) | Meglumine (1) | | |
| Preparation Example 4 | | | Oleic acid (5) | Arginine (1) |
| Preparation Example 5 | Lactic acid (5) | Trometamol (1) | Oleic acid (5) | Arginine (1) |
| Preparation Example 24 | Lactic acid (5) | Arginine (1) | Oleic acid (5) | Meglumine (1) |
| Preparation Example 25 | Lactic acid (5) | Meglumine (1) | Oleic acid (5) | Trometamol (1) |
| Preparation Example 26 | Malic acid (1) | Diethanolamine (10) | Oleic acid (5) | Diethanolamine (10) |

As experimental animals, BALB/c mice (7-week age, male) (purchased from Japan SLC (Shizuoka, JAPAN)) were used.

The BALB/c mice were fasted overnight, and the mice were given each preparation of Preparation Examples 1 to 5 and 24 to 26 comprising FITC-labeled dextran in the adjusted amount (12.5 mg/kg as the FITC-labeled dextran amount) orally. Blood was collected from the inferior vena cava of each mouse at 1 hour after the administration of each preparation, the collected blood was placed at ordinary temperature for about 30 minutes and centrifuged (4°C, 15 min, 1,500 x g) to collect the serum, and the serum was frozen and stored at - 80°C. The fluorescence intensity of the FITC-labeled dextran in serum was measured by a microplate reader (Tecan Infinite F200 pro, Tecan, Mannedorf, Switzerland) (Excitation wavelength: 485 nm, Fluorescence wavelength: 535 nm).

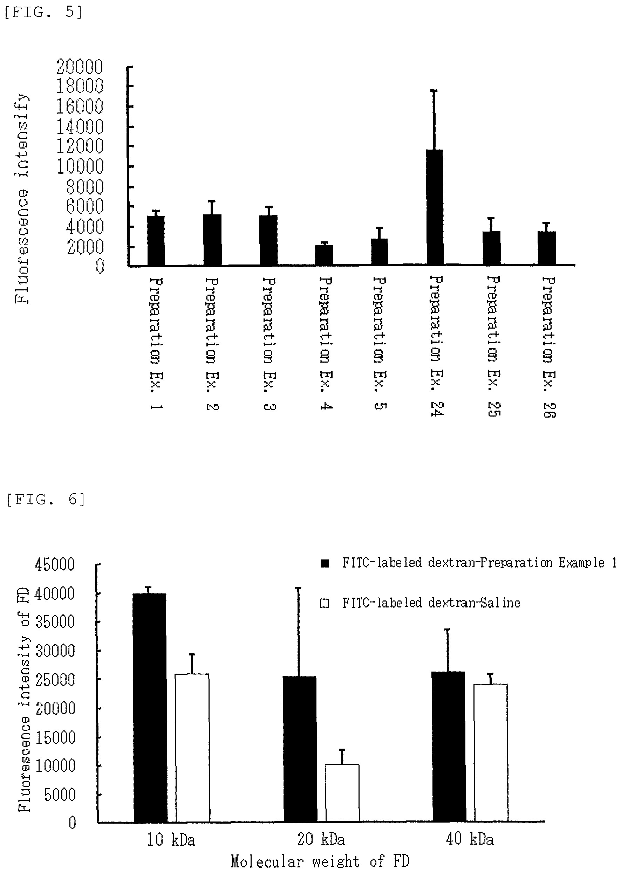

The result is shown in FIG. 5. FIG. 5 shows that the preparation of Preparation Example 24 had the highest fluorescence intensity, followed by the preparations of Preparation Examples 1 to 3. On the other hand, the preparation of Preparation Example 4 comprising only a combination of oleic acid and arginine did not have good fluorescence intensity.

### Example 6: Evaluation of migration ability of poorly absorbable drug into blood when administered orally (2)

In this study, three types of dextrans with different average molecular weights were used and the migration ability of each drug into blood was studied according to the following procedures. Dextrans with average molecular weights of 10000 (10 kDa), 20000 (20 kDa) and 40000 (40 kDa) were purchased from Sigma-Aldrich (MO, USA).

According to the method of Example 1, the preparation of Preparation Example 1 comprising dextran with an average molecular weight of 10 kDa, 20 kDa or 40 kDa as poorly absorbable drug was prepared. Specifically, the ingredients were mixed in the amounts (molar ratios) shown in the above Table 3 to prepare each ionic liquid formulation, FITC-labeled dextran was added into the prepared ionic liquid formulation to adjust the concentration of the ionic liquid to 0.125 M/mL, and the mixture was heated and dissolved at 65°C for 10 minutes to prepare each preparation.

As a control, the preparations in which each dextran is dissolved in saline were used.

As experimental animals, BALB/c mice (7-week age, male) (purchased from Japan SLC (Shizuoka, JAPAN)) were used. According to the test method of Example 5, the fluorescence intensity of FITC-labeled dextran in serum was measured.

The result is shown in FIG. 6. FIG. 6 shows that the dextrans with average molecular weights of 10 kDa and 20 kDa used in Preparation Example 1 had significant fluorescence intensity compared to the control, but the dextran with an average molecular weight of 40kDa did not have significant fluorescence intensity compared to the control.

### INDUSTRIAL APPLICABILITY

According to the present invention, even a poorly absorbable drug that is difficult to be administered orally can be prepared as an oral preparation by markedly improving the absorbability of the drug. Also, according to the present invention, preparations with better bioavailability than conventional oral preparations can be prepared.

## Claims

1. A composition comprising an ionic liquid and a poorly absorbable drug, wherein the ionic liquid is prepared from an anion and a cation, the anion is an organic acid having 3 to 7 carbon atoms, and the cation is selected from the group consisting of arginine, meglumine, trometamol, and diethanolamine.

2. The composition according to claim 1, wherein the poorly absorbable drug is a water-soluble compound with a molecular weight of 3000 to 20000.

3. The composition according to claim 1 or 2, wherein the poorly absorbable drug is GLP-1 agonist.

4. The composition according to any one of claims 1 to 3, wherein the organic acid is one or more selected from the group consisting of lactic acid, citric acid, tartaric acid, malic acid, succinic acid, and benzoic acid.

5. The composition according to any one of claims 1 to 4, which further comprises one or more water-soluble macromolecules selected from the group consisting of hydroxypropyl cellulose, hydroxypropyl methylcellulose, and polyvinylpyrrolidone.
